# EUROPEAN PATENT SPECIFICATION

(11) **EP 1 599 191 B1**
(45) Date of publication and mention of the grant of the patent: **21.04.2010**
(21) Application number: 04714997.6
(22) Date of filing: 26.02.2004
(51) Int. Cl.: A61K 9/28, A61K 31/663

(54) **PHARMACEUTICAL COMPOSITION OF ALENDRONIC ACID, SALTS OR ESTERS THEREOF, AND A PROCESS FOR ITS PREPARATION**
PHARMAZEUTISCHE ZUSAMMENSETZUNG VON ALENDRONSÄURE, SALZE UND ESTER DAVON, UND EIN VERFAHREN ZU DEREN HERSTELLUNG
COMPOSITION PHARMACEUTIQUE A BASE D'ACIDE ALENDRONIQUE, DE SELS OU D'ESTERS DE CELUI-CI ET PROCEDE DE PREPARATION DE CETTE COMPOSITION

(30) Priority: 27.02.2003 SI 200300052
(43) Date of publication of application: 30.11.2005
(73) Proprietor: LEK Pharmaceuticals d.d., 1526 Ljubljana (SI)
(72) Inventor: JAKLIC, Miha, Tomaz, 1262 Dol pri Ljubljani (SI); HUMAR, Vlasta, 1242 Stahovica (SI); SUKLJE-DEBELJAK, Helena, 1000 Ljubljana (SI); SIRCA, Judita, 1000 Ljubljana (SI); MOHAR, Milojka, 1230 Domzale (SI)
(86) International application number: PCT/SI2004/000011
(87) International publication number: WO 2004/075828

(56) References cited:
- WO-A-00/45794
- WO-A-01/26633
- WO-A-02/00204
- US-B1- 6 465 017

## Description

### Field of the invention

The present invention belongs to the field of pharmaceutical technology and relates to a pharmaceutical composition for oral administration of alendronic acid, pharmaceutically acceptable salts or esters thereof, which reduces the potential for irritation and erosion of the mucosal tissue and pain in the upper gastrointestinal tract and provides relatively good absorption of the active substance in the gastrointestinal tract.

### Background of the invention

Oral administration of alendronic acid, pharmaceutically acceptable salts or esters thereof is associated with the esophageal irritation and erosion. Absorption of alendronic acid in the gastrointestinal tract is very poor, it is initiated in the stomach and is continued in the small intestine. Thus, for oral administration of alendronic acid, pharmaceutically acceptable salts or esters thereof special pharmaceutical dosage forms are suitable which provide target release of the active substance in the stomach. Therefore, the film coatings which disintegrate in the intestine are not suitable for pharmaceutical compositions containing alendronic acid, its salts or esters. Further, the film coatings comprising hydroxypropyl methylcellulose may become hard and may delay release of the active substance from the tablet core after a prolonged period of storage.

Ingestion of tablets may be aided by a discontinuous wax polish that does not influence the rate of release of the active substance from the tablet core in the stomach. However, such coating does not completely prevent release of the active substance in the esophagus and a contact of the active substance upon the esophageal mucosal tissue.

In case of pharmaceutical compositions which remain in one piece in the stomach, there may occur a high local concentration of the active substance that may cause damage to the gastric mucosa and pain.

### Prior art

Bisphosphonic acids, salts and esters thereof, are bone resorption inhibitors. They are useful in the prevention and treatment of diseases of bone and calcium metabolism such as, for instance, osteoporosis, Paget's disease, malignant hypercalcemia (H. Fleisch, Bisphosphonates in Bone Disease, from the laboratory to the patient, third edition, 1997). The group of bisphopshonates includes alendronate, cimadronate, etidronate, ibandronate, clodronate, medronate, neridronate, oxydronate, olpadronate, pamindronate, pyridronate, risedronate, tiludronate and zoledronate.

Alendronate is poorly absorbed in the gastrointestinal tract following oral administration. Absorption is reduced when alendronate is taken during the meals, especially in the presence of calcium ions, for example, when consuming milk or dairy products. Alendronate may also cause certain upper gastrointestinal adverse effects, especially the esophageal irritation and erosion. Therefore, it is recommended that the patients take alendronate on an empty stomach with a full glass of water, they should fast and remain in upright posture for at least 30 minutes after administration. These adverse effects appear to be more prevalent in patients who do not follow the directions for use.

Different solid pharmaceutical compositions for oral administration of alendronic acid and/or salts or esters thereof are known.

WO 94/12200 describes pharmaceutical compositions for oral administration of bisphosphonates prepared by direct tabletting. The pharmaceutical composition comprises anhydrous lactose as a diluent, a dry binder, a disintegrant and a lubricant, and optionally additional pharmaceutically acceptable ingredients, e.g., flavours and sweeteners.

WO 95/29679 describes the process of wet granulation for the preparation of tablets of the same composition as in the above example.

WO 02/03963 relates to the tablets of alendronate obtained by direct tabletting which comprise two fillers selected from microcrystalline cellulose or pulverised cellulose, calcium hydrogen phosphate, mannitol, modified starches and phoshates or hydrogen phosphates of alkali and alkaline earth metals. Tablets may also comprise a disintegrant, a dry binder and a lubricant.

WO 97/44017 is directed to effervescent pharmaceutical formulations of bisphonatates comprising an acid, a carbonate, a binder and a lubricant, and optionally flavouring agents, colorants and sweeteners.

Pharmaceutical enteric-coated oral dosage forms for release of the bisphosphonate active substances in the intestine are described in the patent applications WO 93/09785, WO 95/08331 and WO 01/82903.

WO 98/56360 discloses oral oval shaped pharmaceutical dosage forms for release in the stomach. The dosage forms comprise a film coating, which facilitates rapid esophageal transit thereby protecting the mouth, buccal cavity, pharynx and esophagus from irritation. The film coating is selected from the following coatings: hydroxypropylmethylcellulose, hydroxypropylcellulose, carboxymethylcellulose, methylcellulose, ethylcellulose, acrylic resins, polyvinylpyrrolidone and gelatine or mixtures thereof.

WO 01/01991 relates to the tablets of bisphosphonates coated with a discontinuous wax polish that makes the tablets easily swallowable because the wax-polished tablet surface is smoother than the uncoated tablet surface.

WO 02/00204 describes a pharmaceutical composition for controlled release of bisphosphonates in the stomach. Said pharmaceutical composition, which swells in the contact with the gastric juices, contains a non-hydrated hydrogel, a superdisintegrant and tannic acid.

WO 00/45794 describes the film coatings for oral pharmaceutical compositions which do not substantially retard the release rate of the active substance from the tablet core in the stomach. The film coating composition comprises microcrystalline cellulose and carrageenan, and a strengthening polymer and/or plasticizer, and may also include other ingredients such as fillers, surfactants, colouring agents and gloss enhancers.

The aim of the present invention is safe and effective delivery of alendronic acid, pharmaceutically acceptable salts or esters thereof, to the absorption site in the gastrointestinal tract.

### Summary of the invention

The present invention relates to novel pharmaceutical composition useful for administering alendronic acid, its salts or esters. The composition of the present invention comprises a rapidly disintegrating tablet core and a film coating comprising microcrystalline cellulose and carrageenan. The film coating of the present invention facilitates ingestion and prevents irritation and erosion of the upper gastrointestinal tract mucosa while maintaing the bioavailability of the active substance.

In another embodiment, the present invention relates to a process for the preparation of pharmaceutical composition of the present invention comprising:
- forming a rapidly disintegrating tablet core by method of direct compression, dry granulation or wet granulation and
- coating the rapidly disintegrating tablet cores with a film coating comprising microcrystalline cellulose and carrageenan.

In dry granulation method the processes of slugging or compaction are used.

### Detailed description of the invention

The present invention relates to a pharmaceutical composition for oral administration of the active substance, which is alendronic acid (4-amino-1-hydroxybutylidene-1,1-bisphosphonic acid), and pharmaceutically acceptable salts or esters thereof.

Pharmaceutically acceptable salts may be selected form the group consisting of alkali metal salts, alkaline earth metal salts or ammonium salts, preferably salts are selected from the group consisting of sodium, potassium, calcium and magnesium salts.

Most preferably, alendronic acid is in a form of monosodium salt trihydrate which is in the present art known by the names alendronate, sodium alendronate or sodium alendronate trihydrate.

The pharmaceutical composition of the present invention comprises from about 5 to about 280 mg of alendronic acid or a pharmaceutically acceptable salt or ester thereof, on an alendronic acid active weight basis.

The pharmaceutical composition of the present invention comprises a rapidly disintegrating tablet core and a film coating comprising microcrystalline cellulose and carrageenan. The novel pharmaceutical composition provides rapid and targeted delivery of the active substance to the stomach. The film coating facilitates ingestion and prevents irritation and erosion of the buccal cavity, pharynx and esophagus and at the same time does not retard the release of the active substance in the stomach, thereby maintaing the bioavailability of the active substance. In the stomach the rapidly disintegrating tablet core of the present invention disintegrates to tiny particles which are distributed throughout the stomach thus reducing local irritation of the gastric mucosa. From the tablet core, decomposed into tiny particles, the active substance is rapidly released, thereby providing optimal conditions for relatively good absorption of the active substance in the gastrointestinal tract.

The pharmaceutical composition of the present invention is a patient-friendly dosage form. The film coating precludes the contact of the active substance upon the buccal, pharyngeal and esophageal mucosa thus preventing the erosion of the mucosal tissue which may occur upon ingestion of pharmaceutical compositions containing alendronic acid, salts or esters thereof. The film coating, comprising microcrystalline cellulose and carrageenan, hydrates in contact with water and becomes slippery due to the chemical and physical properties of carrageenan, thus facilitating ingestion and rapid and reliable transit of the tablet through the esophagus to the stomach. A shorter transit time of the pharmaceutical composition through the esophagus to the stomach additionally reduces the potential risk of the erosion. A possibility that a pharmaceutical composition is stopped in the esophagus is reduced owing to a gliding coating. Namely, if the active substance was released in the esophagus, high local concentrations would occur which might cause irritation and erosion of the esophageal mucosa. Because of the film coating the pharmaceutical composition of the present invention remains in one piece approximately for 60 seconds after its contact with water, which is sufficient to enter the stomach and not decompose in the esophagus.

The rapidly disintegrating tablet core of the pharmaceutical composition of the present invention may comprise one or more fillers, preferably only one filler, and one or more pharmaceutically acceptable excipients which may be selected from the group consisting of disintegrants, glidants and lubricants and other pharmaceutically acceptable excipients known in the pharmaceutical composition art, e.g., sweetening agents and flavouring agents.

The selection of the suitable combination of a filler and a disintegrant provides rapid disintegration of the pharmaceutical composition in the gastric juices. We have found that rapidly disintegrating tablet core disintegrates within 20 seconds after water penetrates the film coating. The rapidly disintegrating tablet core disintegrates to tiny particles which are (due to fast disintegration) distributed throughout the stomach. In this way local irritation of the gastric mucosa is minimized in comparison to pharmaceutical compositions which disintegrate slower in the stomach and thus causing strong local irritation which may also cause erosion of the mucosal tissue and pain in the stomach. The pharmaceutical composition of the present invention reduces the potential for occurrence of adverse effects also in patients who do not accurately follow the directions for administration of the alendronate tablets.

Rapid disintegration of the tablet core into tiny particles provides rapid dissolution of the active substance in the stomach. Thus, a sufficiently high concentration of active substance is provided which is needed for optimum absorption in the stomach and in the small intestine.

We have found that within 15 minutes already about 90% of active substance is dissolved from the pharmaceutical composition of the present invention. Rapid dissolution of the active substance provides a faster onset of absorption and faster passage of the active substance from the stomach into the duodenum and the small intestine.

A filler of the rapidly disintegrating tablet core of the present invention may be selected from the group consisting of microcrystalline cellulose, silicified microcrystalline cellulose, powdered cellulose, lactose, mannitol, sorbitol, calcium phosphate and calcium hydrogen phosphate. Preferably, the filler is microcrystalline cellulose or silicified microcrystalline cellulose. The rapidly disintegrating tablet core comprises from about 40 to about 99% by weight of filler.

A disintegrant of the rapidly disintegrating tablet core of the present invention may be selected from the group consisting of sodium croscarmellose, starch, pregelatinized starch, sodium carboxymethyl starch, calcium carboxymethylcellulose, sodium carboxymethylcellulose, low-substituted hydroxypropylcellulose, alginic acid, pectinic acid, polyvinylpyrrolidone, cross-linked polyvinylpyrrolidone, highly-dispersed silicon dioxide and sodium alginate. Preferably the disintegrant is sodium croscarmellose or sodium carboxymethyl starch. The rapidly disintegrating tablet core comprises from about 0 to about 10% by weight of disintegrant.

A glidant of the rapidly disintegrating tablet core of the present invention may be selected from the group consisting of high-dispersed silicon dioxide, talc, magnesium stearate, calcium stearate, aluminium stearate, palmitic acid, stearic acid, stearol, cetanol and polyethylene glycol. Preferably, the glidant is silicon dioxide. The rapidly disintegrating tablet core comprises from about 0 to about 5% by weight of glidant.

A lubricant of the rapidly disintegrating tablet core of the present invention may be selected from the group consisting of stearic acid, magnesium stearate, calcium stearate, aluminium stearate, zinc stearate, sodium lauryl sulfate, sodium stearyl fumarate, talc, siliconized talc, hydrogenated vegetable oil and polyethylene glycols. Preferably, the lubricant is magnesium stearate or sodium stearyl fumarate. The rapidly disintegrating tablet core comprises from about 0 to about 5% by weight of lubricant.

The film coating of the present invention comprises microcrystalline cellulose, carrageenan, and at least one of a strengthening polymer and/or a plasticizer. Preferably, carageenan is iota carrageenan.

The mixture for the film coating of the present invention may be prepared or a suitable commercially available dry film coating mixture is used, e.g., Lustre Clear^{™}. Lustre Clear^{™} is the tradename for the coating system in the form of the dry powder manufactured by FMC BioPolymer. A coating dispersion is prepared by dispersing Lustre Clear^{™} in water.

A suitable film forming amount of carrageenan is generally in the range of about 9% to about 25%, preferably in the range of about 10% to about 20% by the weight of the dry film coating mixture.
A ratio of microcrystalline cellulose to carrageenan in the dry film coating mixture is from about 90:10 (w/w) to about 60:40 (w/w), preferably about 70:30 (w/w).

A strengthening polymer may be selected from the group consisting of hydroxyethylcellulose, hydroxypropylmethylcellulose, hydroxypropylcellulose, ethylcellulose, methylcellulose and polyvinylpyrrolidone, preferably hydroxyethylcellulose, and is used in amounts from about 0.5% to about 30% by the weight of the dry film coating mixture.

A plasticizer may be selected from the group consisting of high molecular weight polyethylene glycols, triacetin, dibutyl sebacate, propylene glycol, sorbitol, glycerin and triethyl citrate, and is used in amounts from about 18 to about 36% by the weight of the dry film coating mixture, preferably from about 31 to about 35 % by the weight of the dry film coating mixture.

The film coating may also comprise other ingredients such as different diluents, surfactants, gloss enhancers.
A diluent may be selected from the group consisting of calcium carbonate, dicalcium phosphate, starch, maltodextrin, lactose and mannitol.
A surfactant may be selected from the group consisting of sodium lauryl sulphate, hydroxylated soy lecithin, polysorbates and block copolymers of propylene oxide and ethylene oxide.
A colouring agent may be selected from the group consisting of aluminium lakes, insoluble pigments, water-soluble dyes, titanium dioxide and talc.
Gloss enhancers may be selected from the group consisting of stearic acid or salts or esters thereof and propylene glycol alginate.

The present invention also relates to the process for the preparation of pharmaceutical composition of the present invention. The process includes forming a rapidly disintegrating tablet core and coating the rapidly disintegrating tablet core with a film coating comprising microcrystalline cellulose and carrageenan.

The rapidly disintegrating tablet cores of the present invention may be prepared by the processes known in pharmaceutical technology. That is by direct tabletting of the mixture of the powders or by tabletting of the granulate prepared by dry or wet granulation. In dry granulation, e.g., the processes of slugging or compaction are used. Preferably, the rapidly disintegrating tablet cores are manufactured by direct compression or tabeletting of the granulate which is prepared by the process of slugging or compacting.

The film coating may be applied onto the rapidly disintegrating tablet cores of the present invention by the processes which are conventional in pharmaceutical technology. Preferably, the film coating is applied by spraying the film coating dispersion. The film coating dispersion is prepared by dispersing the dry film coating mixture in water.

The process for the preparation of the pharmaceutical composition of the present invention may comprise the following steps:
e) preparing mixture of the active substance selected from the group consisting of alendronic acid, esters or salts thereof, one or more fillers and one or more pharmaceutically acceptable excipients selected from the group consisting of disintegrants, glidants and other pharmaceutically acceptable excipients,
b) mixing the mixture and a lubricant,
c) compressing the resulting mixture into the tablets using the method of direct tabletting,
d) film coating of the tablet with the coating comprising microcrystalline cellulose and carrageenan.

The process for the preparation of the pharmaceutical composition of the present invention may comprise the following steps:
a) preparing mixture of the active substance selected from the group consisting of alendronic acid, esters or salts thereof, one or more fillers and one or more pharmaceutically acceptable excipients selected from the group consisting of disintegrants, glidants and other pharmaceutically acceptable excipients,
b) mixing the mixture and a lubricant,
c) compressing the resulting mixture into the compacts,
d) milling the compacts into to the granulate,
e) preparing the mixture of the granulate and a lubricant,
f) compressing the resulting mixture into the tablets
g) film coating of the tablet with the coating comprising microcrystalline cellulose and carrageenan.

The process for the preparation of the pharmaceutical composition of the present invention may comprise the following steps:
a) preparing mixture of the active substance selected from the group consisting of alendronic acid, esters or salts thereof, one or more fillers and one or more pharmaceutically acceptable excipients selected from the group consisting of disintegrants, glidants and other pharmaceutically acceptable excipients,
b) preparing the granulate by the method of wet granulation,
c) preparing the mixture of the granulate and a lubricant,
d) compressing the resulting mixture into the tablets,
e) film coating of the tablet with the coating comprising microcrystalline cellulose and carrageenan.

The pharmaceutical composition of the present invention containing alendronic acid, pharmaceutically acceptable salts or esters thereof is useful in the prevention and treatment of the diseases of bone and calcium metabolism, such as osteoporosis, Paget's disease, malignant hypercalcemia.

The invention is illustrated but not in any way limited by the following examples:

### Example 1:

### Composition of 10 mg film coated tablet:

| | Composition/tablet |
|---|---|
| **Tablet core** | |
| Sodium alendronate trihydrate | 13.050 mg |
| Microcrystalline cellulose | 183.450 mg |
| Sodium carboxymethyl starch | 0.500 mg |
| Silicon dioxide | 2.000 mg |
| Magnesium stearate | 1.000 mg |
| **Film coating** | |
| Lustre Clear^{™} LC103 | 4.000 mg |
| Demineralized water | 40.500 mg |

### Process for the preparation:

Sodium alendronate trihydrate (equivalent to 10 mg of alendronic acid), microcrystalline cellulose, sodium carboxymethyl starch and silicon dioxide were mixed for a short time. The blend was sieved through the screen and mixed. Magnesium stearate was sieved through a screen and added to the basic blend and mixed for a short time. The resulting mixture was compressed into tablets each weighing 200 mg.
Lustre Clear^{™} LC103 was dispersed in water at a temperature of 85°C and stirred during cooling for 60 to 120 minutes. The resulting suspension was sprayed onto the tablet cores in a coating pan.

### Example 2:

### Composition of 35 mg film coated tablet:

| | Composition/tablet |
|---|---|
| **Tablet core** | |
| Sodium alendronate trihydrate | 45.675 mg |
| Silicified microcrystalline cellulose | 150.525 mg |
| Sodium croscarmellose | 0.300 mg |
| Silicon dioxide | 2.000 mg |
| Sodium stearyl fumarate | 1.750 mg |
| **Film coating** | |
| Lustre Clear^{™} LC103 | 4.000 mg |
| Demineralized water | 40.500 mg |

### Process for the preparation:

Sodium alendronate trihydrate (equivalent to 35 mg of alendronic acid), silicified microcrystalline cellulose, sodium croscarmellose and silicon dioxide were mixed for a short time. The blend was sieved through the screen and mixed. Sodium stearyl fumarate was sieved through a screen and added to the basic blend and mixed for a short time. The resulting mixture was compressed into tablets each weighing 200 mg.
Lustre Clear^{™} LC103 was dispersed in water at a temperature of 85°C and stirred during cooling for 60 to 120 minutes. The resulting suspension was sprayed onto the tablet cores in a coating pan.

### Example 3:

### Composition of 70 mg film coated tablet:

| | Composition/tablet |
|---|---|
| **Tablet core** | |
| Sodium alendronate trihydrate | 91.350 mg |
| Microcrystalline cellulose | 261.995 mg |
| Sodium croscarmellose | 0.530 mg |
| Silicon dioxide | 3.500 mg |
| Magnesium stearate | 2.625 mg |
| **Film coating** | |
| Lustre Clear^{™} LC103 | 7.000 mg |
| Demineralized water | 70.800 mg |

### Process for the preparation:

Sodium alendronate trihydrate (equivalent to 70 mg of alendronic acid), microcrystalline cellulose, sodium croscarmellose and silicon dioxide were mixed for a short time. The blend was sieved through the screen and mixed. Magnesium stearate was sieved through a screen and added to the basic blend and mixed for a short time. The resulting mixture was compressed into tablets each weighing 360 mg.
Lustre Clear^{™} LC103 was dispersed in water at a temperature of 85°C and stirred during cooling for 60 to 120 minutes. The resulting suspension was sprayed onto the tablet cores in a coating pan.

### Example 4:

### Composition of 70 mg film coated tablet:

| | Composition/tablet |
|---|---|
| **Tablet core** | |
| Sodium alendronate trihydrate | 91.350 mg |
| Microcrystalline cellulose | 261.995 mg |
| Sodium croscarmellose | 0.530 mg |
| Silicon dioxide | 3.500 mg |
| Magnesium stearate | 2.625 mg |
| **Film coating** | |
| Lustre Clear^{™} LC103 | 5.000 mg |
| Demineralized water | 50.600 mg |

### Process for the preparation:

Sodium alendronate trihydrate (equivalent to 70 mg of alendronic acid), microcrystalline cellulose, sodium croscarmellose and silicon dioxide were mixed for a short time. The blend was sieved through the screen and mixed. Magnesium stearate was sieved through a screen and added to the basic blend and mixed for a short time. The resulting mixture was compressed into tablets each weighing 360 mg.
Lustre Clear^{™} LC103 was dispersed in water at a temperature of 85°C and stirred during cooling for 60 to 120 minutes. The resulting suspension was sprayed onto the tablet cores in a coating pan.

### Example 5:

### Composition of 70 mg film coated tablet:

| | Composition/tablet |
|---|---|
| **Tablet core** | |
| Sodium alendronate trihydrate | 91.350 mg |
| Microcrystalline cellulose | 261.995 mg |
| Sodium croscarmellose | 0.530 mg |
| Silicon dioxide | 3.500 mg |
| Magnesium stearate | 2.625 mg |
| **Film coating** | |
| Lustre Clear^{™} LC103 | 9.000 mg |
| Demineralized water | 91.000 mg |

### Process for the preparation:

Sodium alendronate trihydrate (equivalent to 70 mg of alendronic acid), microcrystalline cellulose, sodium croscarmellose and silicon dioxide were mixed for a short time. The blend was sieved through the screen and mixed. Magnesium stearate was sieved through a screen and added to the basic blend and mixed for a short time. The resulting mixture was compressed into tablets each weighing 360 mg.

Lustre Clear^{™} LC103 was dispersed in water at a temperature of 85°C and stirred during cooling for 60 to 120 minutes. The resulting suspension was sprayed onto the tablet cores in a coating pan.

### Example 6:

### Composition of 70 mg film coated tablet:

| | Composition/tablet |
|---|---|
| **Tablet core** | |
| Sodium alendronate trihydrate | 91.350 mg |
| Microcrystalline cellulose | 261.250 mg |
| Sodium croscarmellose | 1.280 mg |
| Silicon dioxide | 3.500 mg |
| Magnesium stearate | 2.620 mg |
| **Film coating** | |
| Lustre Clear^{™} LC103 | 7.000 mg |
| Demineralized water | 70.800 mg |

### Process for the preparation:

Sodium alendronate trihydrate (equivalent to 70 mg of alendronic acid), microcrystalline cellulose, sodium croscarmellose and silicon dioxide were mixed simultaneously for short time. The blend was sieved through the screen and mixed and ½ of the sieved magnesium stearate was added. The mixture was mixed for short time, compressed into the compacts which were then milled in a hammer-type mill. The remaining ½ of the sieved magnesium stearate was added to the granulate and mixed for short time. The resulting granulate was compressed into tablets each weighing 360 mg.

Lustre Clear^{™} LC103 was dispersed in water at a temperature of 85°C and stirred during cooling for 60 to 120 minutes. The resulting suspension was sprayed onto the tablet cores in a coating pan.

### Example 7

### Dissolution test

The tablet cores without the film coating and the tablet cores with the film coating, prepared by the procedure described in Example 3, were dissolved using USP apparatus 2, 50 rpm, 900 ml of water.

**Table 1: Percent of dissolution of alendronate (mean)**

| t(min) | Percent of dissolution (x̅ ± SD (RSD)) | |
|---|---|---|
| | Tablet cores without the film coating | Tablet cores with the film coating set forth in the invention |
| 5 | 82.5 ± 10.0 (12.1) | 83.3 ± 9.8 (11.8) |
| 15 | 91.4 ± 6.3 (6.9) | 91.9 ± 6.3 (6.9) |
| 30 | 97.6 ± 4.7 (4.8) | 95.4 ± 4.9 (5.1) |

As evident from Table 1, there are no significant differences in the dissolution rate of alendronate from the tablet core without the film coating and the film coated tablet core. Dissolution of the active substance is rapid as already about 95% of the active substance is dissolved in 30 minutes.

## Claims

1. A pharmaceutical composition for oral administration of alendronic acid, pharmaceutically acceptable salts or esters thereof, which comprises:
a) a rapidly disintegrating tablet core comprising the active substance and
b) a film coating comprising microcrystalline cellulose and carrageenan.

2. The pharmaceutical composition according to claim 1 wherein the pharmaceutically acceptable salt of alendronic acid is alendronic acid monosodium salt trihydrate.

3. The pharmaceutical composition according to any one of claims 1 to 2 comprising from 5 to 280 mg of alendronic acid or a pharmaceutically acceptable salt or ester thereof, on an alendronic acid active weight basis.

4. The pharmaceutical composition according to claim 3 comprising 5 mg of alendronic acid or a pharmaceutically acceptable salt or ester thereof, on an alendronic acid active weight basis.

5. The pharmaceutical composition according to claim 3 comprising 10 mg of alendronic acid or a pharmaceutically acceptable salt or ester thereof, on an alendronic acid active weight basis.

6. The pharmaceutical composition according to claim 3 comprising 35 mg of alendronic acid or a pharmaceutically acceptable salt or ester thereof, on an alendronic acid active weight basis.

7. The pharmaceutical composition according to claim 3 comprising 40 mg of alendronic acid or a pharmaceutically acceptable salt or ester thereof, on an alendronic acid active weight basis.

8. The pharmaceutical composition according to claim 3 comprising 70 mg of alendronic acid or a pharmaceutically acceptable salt or ester thereof, on an alendronic acid active weight basis.

9. The pharmaceutical composition according to claim 3 comprising 140 mg of alendronic acid or a pharmaceutically acceptable salt or ester thereof, on an alendronic acid active weight basis.

10. The pharmaceutical composition according to claim 3 comprising 280 mg of alendronic acid or a pharmaceutically acceptable salt or ester thereof, on an alendronic acid active weight basis.

11. The pharmaceutical composition according to any one of claims 1 to 10 wherein the rapidly disintegrating tablet core, in addition to the active substance, comprises one or more fillers and one or more pharmaceutically acceptable excipients selected from the group consisting of disintegrants, glidants, lubricants and other pharmaceutically acceptable excipients.

12. The pharmaceutical composition according to claim 11 wherein the rapidly disintegrating tablet core comprises only one filler.

13. The pharmaceutical composition according to any one of claims 11 to 12 wherein the filler is selected from the group consisting of microcrystalline cellulose, silicified microcrystalline cellulose powdered cellulose, lactose, mannitol, sorbitol, calcium phosphate and calcium hydrogen phosphate.

14. The pharmaceutical composition according to claim 13 wherein the filler is microcrystalline cellulose.

15. The pharmaceutical composition according to claim 13 wherein the filler is silicified microcrystalline cellulose.

16. The pharmaceutical composition according to any one of claims 11 to 15 wherein the disintegrant is selected from the group consisting of sodium croscarmellose, starch, pregelatinized starch, sodium carboxymethyl starch, calcium carboxymethylcellulose, sodium carboxymethylcellulolose low-substituted hydroxypropylcellulose, alginic acid, pectinic acid, polyvinylpyrrolidone, cross-linked polyvinylpyrrolidone, high-dispersed silicon dioxide and sodium alginate.

17. The pharmaceutical composition according to claim 16 wherein the disintegrant is sodium croscarmellose.

18. The pharmaceutical composition according to any one of claims 11 to 17 wherein the glidant is selected from the group consisting of high-dispersed silicon dioxide, talc, magnesium stearate, calcium stearate, aluminium stearate, palmitic acid, stearic acid, stearol, cetanol and polyethylene glycol.

19. The pharmaceutical composition according to claim 18 wherein the glidant is silicon dioxide.

20. The pharmaceutical composition according to any one of claims 11 to 19 wherein the lubricant is selected from the group consisting of stearic acid, magnesium stearate, calcium stearate, aluminium stearate, zinc stearate, sodium lauryl sulfate, sodium stearyl fumarate, talc, siliconized talc, hydrogenated vegetable oil and polyethylene glycols.

21. The pharmaceutical composition according to claim 20 wherein the lubricant is magnesium stearate.

22. The pharmaceutical composition according to any one of claims 1 to 21 wherein the film coating, in addition to microcrystalline cellulose and carrageenan, comprises one or more pharmaceutically acceptable excipients selected from the group consisting of strengthening polymers, plasticizers, diluents, surfactants, colorants and gloss enhancers.

23. The pharmaceutical composition according to any one of claims 1 to 22 **characterized in that** the rapidly disintegrating tablet core is obtained using the process of direct compression.

24. The pharmaceutical composition according to any one of claims 1 to 22 **characterized in that** the rapidly disintegrating tablet core is obtained using the process of tabletting the granulate.

25. The pharmaceutical composition according to claim 24 **characterized in that** the granulate is obtained by the process of dry granulation.

26. The pharmaceutical composition according to claim 24 **characterized in that** the granulate is obtained by the process of wet granulation.

27. The pharmaceutical composition according to an one of claims 1 to 26 **characterized in that a** film coating is applied onto the rapidly disintegrating tablet core by spraying the coating dispersion.

28. The use of alendronic acid, pharmaceutically acceptable salts or esters thereof, for the preparation of the pharmaceutical composition as claimed in any one of claims 1 to 27 useful in the prevention and treatment of the diseases of bone and calcium metabolism.

29. The use of alendronic acid, pharmaceutically acceptable salts or esters thereof, for the preparation of the pharmaceutical composition as claimed in any one of claims 1 to 27 useful in the prevention and treatment of osteoporosis.

30. The process for the preparation of the pharmaceutical composition which comprises the following steps:
a) preparing mixture of the active substance selected from the group consisting of alendronic acid, esters or salts thereof, one or more fillers and one or more pharmaceutically acceptable excipients selected from the group consisting of disintegrants, glidants and other pharmaceutically acceptable excipients,
b) mixing the mixture with a lubricant,
c) compressing the resulting mixture into the tablets using the method of direct tabletting,
d) film coating of the tablet with the coating comprising microcrystalline cellulose and carrageenan.

31. The process for the preparation of the pharmaceutical composition which comprises the following steps:
a) preparing mixture of the active substance selected from the group consisting of alendronic acid, esters or salts thereof, one or more fillers and one or more pharmaceutically acceptable excipients selected from the group consisting of disintegrants, glidants and other pharmaceutically acceptable excipients,
b) mixing the mixture with a lubricant,
c) compressing the resulting mixture into the compacts,
d) milling the compacts into to the granulate,
e) preparing the mixture of the granulate and a lubricant,
f) compressing the resulting mixture into the tablets,
g) film coating of the tablet with the coating comprising microcrystalline cellulose and carrageenan.

32. The process for the preparation of the pharmaceutical composition which comprises the following steps:
a) preparing mixture of the active substance selected from the group consisting of alendronic acid, esters or salts thereof, one or more fillers and one or more pharmaceutically acceptable excipients selected from the group consisting of disintegrants, glidants and other pharmaceutically acceptable excipients,
b) preparing the granulate by the method of wet granulation,
c) preparing.the mixture of the granulate and a lubricant,
d) compressing the resulting mixture into the tablets,
e) film coating of the tablet with the coating comprising microcrystalline cellulose and carrageenan.

33. The process for the preparation of the pharmaceutical composition as claimed in any one of claims 30 to 32 **characterized in that** the pharmaceutical compositions comprises from 5 to 280 mg of alendronic acid or a pharmaceutically acceptable salt or ester thereof, on an alendronic acid active weight basis.

34. The process for the preparation of the pharmaceutical composition according to any one of claims 30 to 33 **characterized in that** the rapidly disintegrating tablet core of the pharmaceutical composition comprises only one filler.

35. The process for the preparation of pharmaceutical composition according to any one of claims 30 to 34 **characterized in that** the filler is selected from the group consisting of microcrystalline cellulose, silicified microcrystalline Cellulose, powdered cellulose, lactose, mannitol, sorbitol, calcium phosphate and calcium hydrogen phosphate.

36. The process for the preparation of pharmaceutical composition according to claim 35 **characterized in that** the filler is microcrystalline cellulose.

37. The process for the preparation of the pharmaceutical composition according to claim 35 **characterized in that** the filler is silicified microcrystalline cellulose.

38. The process for the preparation of the pharmaceutical composition according to any one of claims 30 to 37 **characterized in that** the disintegrant is selected from the group consisting of sodium croscarmellose, starch, pregelatinized starch, sodium carboxymethyl starch, calcium carboxymethylcellulose, sodium carboxymethylcellulose, low-substituted hydroxypropycellulose, alginic acid, pectinic acid, polyvinylpyrrolidone, high-dispersed silicon dioxide and sodium alginate.

39. The process for the preparation of the pharmaceutical composition according to claim 38 **characterized in that** the disintegrant is sodium croscarmellose.

40. The process for the preparation of the pharmaceutical composition according to any one of claims 30 to 39 **characterized in that** the glidant is selected from the group consisting of high-dispersed silicon dioxide, talc, magnesium stearate, calcium stearate, aluminium stearate, palmitic acid, stearic acid, stearol, cetanol and polyethylene glycol.

41. The process for the preparation of the pharmaceutical composition according to claim 40 **characterized in that** the glidant is silicon dioxide.

42. The process for the preparation of the pharmaceutical composition according to any one of claims 30 to 41 **characterized in that** the lubricant is selected from the group consisting of stearic acid, magnesium stearate, calcium stearate, aluminium stearate, zinc stearate, sodium lauryl sulfate, sodium stearyl fumarate, talc, siliconized talc, hydrogenated vegetable oil and polyethylene glycols.

43. The process for the preparation of the pharmaceutical composition according to claim 42 **characterized in that** the lubricant is magnesium stearate.

44. The process for the preparation of the pharmaceutical composition according to any one of claims 30 to 43 **characterized in that** the film coating, in addition to microcrystalline cellulose and carrageenan, comprises one or more pharmaceutically acceptable excipients selected from the group consisting of strengthening polymers, plasticizers, diluents, surfactants, colouring agents and gloss enhancers.

## Patentansprüche

1. Pharmazeutische Zusammensetzung für orale Verabreichung von Alendronsäure, pharmazeutisch annehmbarer Salze oder Ester davon, umfassend:
a) einen schnell zerfallenden Tablettenkern, umfassend den Wirkstoff, und
b) eine Filmbeschichtung, umfassend mikrokristalline Cellulose und Carrageen.

2. Pharmazeutische Zusammensetzung nach Anspruch 1, wobei das pharmazeutisch annehmbare Salz von Alendronsäure Alendronsäure-Mononatriumsalztrihydrat ist.

3. Pharmazeutische Zusammensetzung nach einem der Ansprüche 1 bis 2, umfassend 5 bis 280 mg Alendronsäure oder pharmazeutisch annehmbares Salz oder pharmazeutisch annehmbaren Ester davon, bezogen auf das Gewicht der aktiven Alendronsäure.

4. Pharmazeutische Zusammensetzung nach Anspruch 3, umfassend 5 mg Alendronsäure oder pharmazeutisch annehmbares Salz oder pharmazeutisch annehmbaren Ester davon, bezogen auf das Gewicht der aktiven Alendronsäure.

5. Pharmazeutische Zusammensetzung nach Anspruch 3, umfassend 10 mg Alendronsäure oder pharmazeutisch annehmbares Salz oder pharmazeutisch annehmbaren Ester davon, bezogen auf das Gewicht der aktiven Alendronsäure.

6. Pharmazeutische Zusammensetzung nach Anspruch 3, umfassend 35 mg Alendronsäure oder pharmazeutisch annehmbares Salz oder pharmazeutisch annehmbaren Ester davon, bezogen auf das Gewicht der aktiven Alendronsäure.

7. Pharmazeutische Zusammensetzung nach Anspruch 3, umfassend 40 mg Alendronsäure oder pharmazeutisch annehmbares Salz oder pharmazeutisch annehmbaren Ester davon, bezogen auf das Gewicht der aktiven Alendronsäure.

8. Pharmazeutische Zusammensetzung nach Anspruch 3, umfassend 70 mg Alendronsäure oder pharmazeutisch annehmbares Salz oder pharmazeutisch annehmbaren Ester davon, bezogen auf das Gewicht der aktiven Alendronsäure.

9. Pharmazeutische Zusammensetzung nach Anspruch 3, umfassend 140 mg Alendronsäure oder pharmazeutisch annehmbares Salz oder pharmazeutisch annehmbaren Ester davon, bezogen auf das Gewicht der aktiven Alendronsäure.

10. Pharmazeutische Zusammensetzung nach Anspruch 3, umfassend 280 mg Alendronsäure oder pharmazeutisch annehmbares Salz oder pharmazeutisch annehmbaren Ester davon, bezogen auf das Gewicht der aktiven Alendronsäure.

11. Pharmazeutische Zusammensetzung nach einem der Ansprüche 1 bis 10, wobei der schnell zerfallende Tablettenkern zusätzlich zu dem Wirkstoff einen oder mehrere Füllstoffe und einen oder mehrere pharmazeutisch annehmbare Hilfsstoffe ausgewählt aus der Gruppe bestehend aus Sprengmittel, Fließregulierungsmittel, Gleitmittel und anderen pharmazeutisch annehmbaren Hilfsstoffen umfasst.

12. Pharmazeutische Zusammensetzung nach Anspruch 11, wobei der schnell zerfallenden Tablettenkern nur einen Füllstoff umfasst.

13. Pharmazeutische Zusammensetzung nach einem der Ansprüche 11 bis 12, wobei der Füllstoff aus der Gruppe bestehend aus mikrokristalliner Cellulose, verkieselter mikrokristalliner Cellulose, Cellulosepulver, Lactose, Mannit, Sorbit, Calciumphosphat und Calciumhydrogenphosphat ausgewählt ist.

14. Pharmazeutische Zusammensetzung nach Anspruch 13, wobei der Füllstoff mikrokristalline Cellulose ist.

15. Pharmazeutische Zusammensetzung nach Anspruch 13, wobei der Füllstoff verkieselte mikrokristalline Cellulose ist.

16. Pharmazeutische Zusammensetzung nach einem der Ansprüche 11 bis 15, wobei das Sprengmittel aus der Gruppe bestehend aus Croscarmellose-Natrium, Stärke, vorgelatinisierter Stärke, Carboxymethylstärke-Natrium, Carboxymethylcellulose-Calcium, Carboxymethylcellulose-Natrium, niedersubstituierter Hydroxypropylcellulose, Alginsäure, Pectinsäure, Polyvinylpyrrolidon, vernetztem Polyvinylpyrrolidon, hochdispersem Siliciumdioxid und Natriumalginat ausgewählt ist.

17. Pharmazeutische Zusammensetzung nach Anspruch 16, wobei das Sprengmittel Croscarmellose-Natrium ist.

18. Pharmazeutische Zusammensetzung nach einem der Ansprüche 11 bis 17, wobei das Fließregulierungsmittel aus der Gruppe bestehend aus hochdispersem Siliciumdioxid, Talkum, Magnesiumstearat, Calciumstearat, Aluminiumstearat, Palmitinsäure, Stearinsäure, Stearol, Cetanol und Polyethylenglykol ausgewählt ist.

19. Pharmazeutische Zusammensetzung nach Anspruch 18, wobei das Fließregulierungsmittel Siliciumdioxid ist.

20. Pharmazeutische Zusammensetzung nach einem der Ansprüche 11 bis 19, wobei das Gleitmittel aus der Gruppe bestehend aus Stearinsäure, Magnesiumstearat, Calciumstearat, Aluminiumstearat, Zinkstearat, Natriumlaurylsulfat, Natriumstearylfumarat, Talkum, silikonisiertem Talkum, hydriertem Pflanzenöl und Polyethylenglykolen ausgewählt ist.

21. Pharmazeutische Zusammensetzung nach Anspruch 20, wobei das Gleitmittel Magnesiumstearat ist.

22. Pharmazeutische Zusammensetzung nach einem der Ansprüche 1 bis 21, wobei die Filmbeschichtung zusätzlich zu mikrokristalliner Cellulose und Carrageen einen oder mehrere pharmazeutisch annehmbare Hilfsstoffe, ausgewählt aus der Gruppe bestehend aus verstärkenden Polymeren, Weichmachern, Verdünnungsmitteln, Tensiden, Färbungsmitteln und Glanzverstärkern umfasst.

23. Pharmazeutische Zusammensetzung nach einem der Ansprüche 1 bis 22, **dadurch gekennzeichnet, dass** der schnell zerfallende Tablettenkern durch das Verfahren der direkten Verpressung hergestellt wird.

24. Pharmazeutische Zusammensetzung nach einem der Ansprüche 1 bis 22, **dadurch gekennzeichnet, dass** der schnell zerfallende Tablettenkern durch das Verfahren der Granulattablettierung hergestellt wird.

25. Pharmazeutische Zusammensetzung nach Anspruch 24, **dadurch gekennzeichnet, dass** das Granulat durch das Verfahren der Trockengranulation hergestellt wird.

26. Pharmazeutische Zusammensetzung nach Anspruch 24, **dadurch gekennzeichnet, dass** das Granulat durch das Verfahren der Feuchtgranulation hergestellt wird.

27. Pharmazeutische Zusammensetzung nach einem der Ansprüche 1 bis 26, **dadurch gekennzeichnet, dass** eine Filmbeschichtung auf den schnell zerfallenden Tablettenkern durch Sprühen der Beschichtungsdispersion aufgezogen wird.

28. Verwendung von Alendronsäure, pharmazeutisch annehmbarer Salze oder Ester davon zur Herstellung pharmazeutischer Zusammensetzungen, wie in einem der Ansprüche 1 bis 27 beansprucht, die brauchbar zur Vorbeugung und Behandlung von Erkrankungen der Knochen und Störungen des Calciumstoffwechsels sind.

29. Verwendung von Alendronsäure, pharmazeutisch annehmbarer Salze oder Ester davon zur Herstellung der pharmazeutischen Zusammensetzung, wie in einem der Ansprüche 1 bis 27 beansprucht, die brauchbar zur Vorbeugung und Behandlung von Osteoporose ist.

30. Verfahren zur Herstellung von pharmazeutischer Zusammensetzung, umfassend die folgenden Schritte:
a) Herstellen von Mischung des Wirkstoffs ausgewählt aus der Gruppe bestehend aus Alendronsäure, Ester oder Salzen davon, einem oder mehrerer Füllstoffe und einem oder mehrerer pharmazeutisch annehmbarer Hilfsstoffe, ausgewählt aus der Gruppe bestehend aus Sprengmitteln, Fließregulierungsmitteln und anderer pharmazeutisch annehmbarer Hilfsstoffe,
b) Mischen der Mischung mit einem Gleitmittel,
c) Verpressen der entstandenen Mischung in Tabletten nach dem Verfahren der direkten Tablettierung,
d) Filmbeschichten der Tablette mit der Beschichtung bestehend aus mikrokristalliner Cellulose und Carrageen.

31. Verfahren zur Herstellung von pharmazeutischer Zusammensetzung, umfassend die folgenden Schritte:
a) Herstellen von Mischung des Wirkstoffs ausgewählt aus der Gruppe bestehend aus Alendronsäure, Ester oder Salzen davon, einem oder mehrerer Füllstoffe und einem oder mehrerer pharmazeutisch annehmbarer Hilfsstoffe, ausgewählt aus der Gruppe bestehend aus Sprengmitteln, Fließregulierungsmitteln und anderer pharmazeutisch annehmbarer Hilfsstoffe,
b) Mischen der Mischung mit einem Gleitmittel,
c) Verpressen der entstandenen Mischung zu Presslingen,
d) Mahlen der Presslinge zum Granulat,
e) Herstellen der Mischung aus Granulat und Gleitmittel,
f) Verpressen der resultierenden Mischung zu den Tabletten,
g) Filmbeschichten der Tablette mit der Beschichtung bestehend aus mikrokristalliner Cellulose und Carrageen.

32. Verfahren zur Herstellung von pharmazeutischer Zusammensetzung, umfassend die folgenden Schritte:
a) Herstellen von Mischung des Wirkstoffs ausgewählt aus der Gruppe bestehend aus Alendronsäure, Ester oder Salzen davon, einem oder mehrerer Füllstoffe und einem oder mehrerer pharmazeutisch annehmbarer Hilfsstoffe, ausgewählt aus der Gruppe bestehend aus Sprengmitteln, Fließregulierungsmittein und anderer pharmazeutisch annehmbarer Hilfsstoffe,
b) Herstellen des Granulats nach dem Verfahren der Feuchtgranulation,
c) Herstellen der Mischung aus Granulat und Gleitmittel,
d) Verpressen der resultierenden Mischung zu den Tabletten,
e) Filmbeschichten der Tablette mit der Beschichtung bestehend aus mikrokristalliner Cellulose und Carrageen.

33. Verfahren zur Herstellung von pharmazeutischer Zusammensetzung wie in einem der Ansprüche 30 bis 32 beansprucht, **dadurch gekennzeichnet, dass** die pharmazeutische Zusammensetzung von 5 bis 280 mg Alendronsäure oder pharmazeutisch annehmbares Salz oder pharmazeutisch annehmbaren Ester davon umfasst, bezogen auf das Gewicht der aktiven Alendronsäure.

34. Verfahren zur Herstellung von pharmazeutischer Zusammensetzung nach einem der Ansprüche 30 bis 33, **dadurch gekennzeichnet, dass** der schnell zerfallende Tablettenkern der pharmazeutischen Zusammensetzung nur einen Füllstoff umfasst.

35. Verfahren zur Herstellung von pharmazeutischer Zusammensetzung nach einem der Ansprüche 30 bis 34, **dadurch gekennzeichnet, dass** der Füllstoff aus der Gruppe bestehend aus mikrokristalliner Cellulose, verkieselter mikrokristalliner Cellulose, Cellulosepulver, Lactose, Mannit, Sorbit, Calciumphosphat und Calciumhydrogenphosphat ausgewählt wird.

36. Verfahren zur Herstellung von pharmazeutischer Zusammensetzung nach Anspruch 35, **dadurch gekennzeichnet, dass** der Füllstoff mikrokristalline Cellulose ist.

37. Verfahren zur Herstellung von pharmazeutischer Zusammensetzung nach Anspruch 35, **dadurch gekennzeichnet, dass** der Füllstoff verkieselte mikrokristalline Cellulose ist.

38. Verfahren zur Herstellung von pharmazeutische Zusammensetzung nach einem der Ansprüche 30 bis 37, **dadurch gekennzeichnet, dass** das Sprengmittel aus der Gruppe bestehend aus Croscarmellose-Natrium, Stärke, vorgelatinisierter Stärke, Carboxymethylstärke-Natrium, Carboxymethylcellulose-Calcium, Carboxymethylcellulose-Natrium, niedersubstituierter Hydroxypropylcellulose, Alginsäure, Pectinsäure, Polyvinylpyrrolidon, hochdispersem Siliciumdioxid und Natriumalginat ausgewählt wird.

39. Verfahren zur Herstellung von pharmazeutischer Zusammensetzung nach Anspruch 38, **dadurch gekennzeichnet, dass** das Sprengmittel Croscarmellose-Natrium ist.

40. Verfahren zur Herstellung von pharmazeutischer Zusammensetzung nach einem der Ansprüche 30 bis 39, **dadurch gekennzeichnet, dass** das Fließregulierungsmittel aus der Gruppe bestehend aus hochdispersem Siliciumdioxid, Talkum, Magnesiumstearat, Calciumstearat, Aluminiumstearat, Palmitinsäure, Stearinsäure, Stearol, Cetanol und Polyethylenglykol ausgewählt wird.

41. Verfahren zur Herstellung von pharmazeutischer Zusammensetzung nach Anspruch 40, **dadurch gekennzeichnet, dass** das Fließregulierungsmittel Siliciumdioxid ist.

42. Verfahren zur Herstellung von pharmazeutischer Zusammensetzung nach einem der Ansprüche 30 bis 41, **dadurch gekennzeichnet, dass** das Gleitmittel aus der Gruppe bestehend aus Stearinsäure, Magnesiumstearat, Calciumstearat, Aluminiumstearat, Zinkstearat, Natriumlaurylsulfat, Natriumstearylfumarat, Talkum, silikonisiertem Talkum, hydriertem Pflanzenöl und Polyethylenglykolen ausgewählt wird.

43. Verfahren zur Herstellung von pharmazeutischer Zusammensetzung nach Anspruch 42, **dadurch gekennzeichnet, dass** das Gleitmittel Magnesiumstearat ist.

44. Verfahren zur Herstellung von pharmazeutischer Zusammensetzung nach einem der Ansprüche 30 bis 43, **dadurch gekennzeichnet, dass** die Filmbeschichtung, zusätzlich zu mikrokristalliner Cellulose und Carrageen einen oder mehrere pharmazeutisch annehmbare Hilfsstoffe ausgewählt aus der Gruppe bestehend aus verstärkenden Polymeren, Weichmachern, Verdünnungsmittel, Tensiden, Färbungsmitteln und Glanzverstärkern umfasst

## Revendications

1. Composition pharmaceutique pour administration orale d'acide alendronique, de ses sels ou esters pharmaceutiquement acceptables, comprenant :
a) un noyau de comprimé se désagrégeant rapidement comprenant la substance active et
b) un pelliculage qui comprend de la cellulose microcristalline et de la carraghénane.

2. Composition pharmaceutique selon la revendication 1, **caractérisée en ce que** le sel pharmaceutiquement acceptable d'acide alendronique est sous forme d'alendronate monosodique trihydraté.

3. Composition pharmaceutique selon l'une des revendications 1 à 2, comprenant de 5 à 280 mg d'acide alendronique ou l'un de ses sels ou esters pharmaceutiquement acceptables, sur base du poids actif d'acide alendronique.

4. Composition pharmaceutique selon la revendication 3, comprenant 5 mg d'acide alendronique ou un de ses sels ou esters pharmaceutiquement acceptables, sur base du poids actif d'acide alendronique.

5. Composition pharmaceutique selon la revendication 3, comprenant 10 mg d'acide alendronique ou un de ses sels ou esters pharmaceutiquement acceptables, sur base du poids actif d'acide alendronique.

6. Composition pharmaceutique selon la revendication 3, comprenant 35 mg d'acide alendronique ou un de ses sels ou esters pharmaceutiquement acceptables, sur base du poids actif d'acide alendronique.

7. Composition pharmaceutique selon la revendication 3, comprenant 40 mg d'acide alendronique ou un de ses sels ou esters pharmaceutiquement acceptables, sur base du poids actif d'acide alendronique.

8. Composition pharmaceutique selon la revendication 3, comprenant 70 mg d'acide alendronique ou un de ses sels ou esters pharmaceutiquement acceptables, sur base du poids actif d'acide alendronique.

9. Composition pharmaceutique selon la revendication 3, comprenant 140 mg d'acide alendronique ou un de ses sels ou esters pharmaceutiquement acceptables, sur base du poids actif d'acide alendronique.

10. Composition pharmaceutique selon la revendication 3, comprenant 280 mg d'acide alendronique ou un de ses sels ou esters pharmaceutiquement acceptables, sur base du poids actif d'acide alendronique.

11. Composition pharmaceutique d'après l'une des revendications 1 à 10, **caractérisée en ce que** le noyau du comprimé se désagrégeant rapidement comprend, en plus de la substance active, un ou plusieurs charges et un ou plusieurs excipients pharmaceutiquement acceptables sélectionnés à partir du groupe qui consiste en des délitants, des glissants, des lubrifiants et d'autres excipients pharmaceutiquement acceptables.

12. Composition pharmaceutique selon la revendication 11, **caractérisée en ce que** le noyau du comprimé se désagrégeant rapidement comprend une seule charge.

13. Composition pharmaceutique selon l'une des revendications 11 à 12, **caractérisée en ce que** la charge est choisi à partir d'un groupe qui comprend de la cellulose microcristalline, de la cellulose microcristalline silicifiée, de la cellulose en poudre, du lactose, du mannitol, du sorbitol, du phosphate de calcium et du phosphate d'hydrogène de calcium.

14. Composition pharmaceutique selon la revendication 13, **caractérisée en ce que** la matière de remplissage est de la cellulose microcristalline.

15. Composition pharmaceutique selon la revendication 13, **caractérisée en ce que** la charge est de la cellulose microcristalline silicifiée.

16. Composition pharmaceutique selon l'une des revendications 11 à 15, **caractérisée en ce que** le délitant est sélectionné à partir du groupe qui comprend de la croscarmellose sodique, de l'amidon, de l'amidon prégélatinisé, du carboxyméthyl amidon sodique, de la carboxyméthylcellulose calcique, de la carboxyméthylcellulose sodique, de l'hydroxypropylcellulose faiblement substituée, de l'acide alginique, de l'acide pectinique, de la polyvinylpyrrolidone, de la polyvinylpyrrolidone réticulée, du dioxyde de silicium fortement dispersé et de l'alginate sodique.

17. Composition pharmaceutique selon la revendication 16, **caractérisée en ce que** le délitant est de la croscarmellose sodique.

18. Composition pharmaceutique selon l'une des revendications 11 à 17, **caractérisée en ce que** le glissant est sélectionné à partir du groupe qui comprend du dioxyde de silicium fortement dispersé, du talc, du stéarate de magnésium, du stéarate de calcium, du stéarate d'aluminium, de l'acide palmitique, de l'acide stéarique, du stéarol, du cétanol et du polyéthylène glycol.

19. Composition pharmaceutique selon la revendication 18, **caractérisée en ce que** le glissant est du dioxyde de silicium.

20. Composition pharmaceutique selon l'une des revendications 11 à 19, **caractérisée en ce que** le lubrifiant est sélectionné à partir du groupe comprenant de l'acide stéarique, du stéarate de magnésium, du stéarate de calcium, du stéarate d'aluminium, du stéarate de zinc, du laurylsulfate de sodium, du stéaryl-fumarate de sodium, du talc, du talc siliconisé, de l'huile végétale hydrogénée et du polyéthylène glycol.

21. Composition pharmaceutique selon la revendication 20, **caractérisée en ce que** le lubrifiant est du stéarate de magnésium.

22. Composition pharmaceutique selon l'une des revendications 1 à 21, **caractérisée en ce que** le pelliculage, en plus de la cellulose microcristalline et de la carraghénane, comprend un ou plusieurs excipients pharmaceutiquement acceptables sélectionnés à partir du groupe comprenant des polymères de renforcement, des plastifiants, des diluants, des agents tensio-actifs, des colorants et des amplificateurs de brillance.

23. Composition pharmaceutique selon l'une des revendications 1 à 22, **caractérisée en ce que** le noyau de comprimé se désagrégeant rapidement est obtenu au moyen du procédé de compression directe.

24. Composition pharmaceutique selon l'une des revendications 1 à 22, **caractérisée en ce que** le noyau de comprimé se désagrégeant rapidement est obtenu au moyen du procédé de tabletterie de granulé.

25. Composition pharmaceutique selon la revendication 24, **caractérisée en ce que** le granulé est obtenu par le procédé de granulation sèche.

26. Composition pharmaceutique selon la revendication 24, **caractérisée en ce que** le granulé est obtenu par le procédé de granulation humide.

27. Composition pharmaceutique selon l'une des revendications 1 à 26, **caractérisée en ce qu'**un pelliculage est appliqué sur le noyau de comprimé se désagrégeant rapidement en vaporisant le pelliculage.

28. L'utilisation d'acide alendronique, de ses sels ou esters pharmaceutiquement acceptables, pour la préparation de la composition pharmaceutique comme revendiqué dans l'une des revendications 1 à 27 est utile pour la prévention et le traitement de maladies osseuses et du métabolisme du calcium.

29. L'utilisation d'acide alendronique, de ses sels ou esters pharmaceutiquement acceptables, pour la préparation de la composition pharmaceutique comme revendiqué dans l'une des revendications 1 à 27 qui est utile pour la prévention et le traitement de l'ostéoporose.

30. Procédé de préparation de la composition pharmaceutique comprenant les étapes suivantes :
a) la préparation d'un mélange de substance active sélectionnée à partir du groupe qui comprend de l'acide alendronique, ses esters ou sels, une ou plusieurs charges et un ou plusieurs excipients pharmaceutiquement acceptables sélectionnés à partir du groupe comprenant des délitants, des glissants et autres excipients pharmaceutiquement acceptables,
b) le mélange mélangé avec un lubrifiant,
c) la compression du mélange résultant en comprimés au moyen de la méthode de tabletterie directe,
d) le pelliculage du comprimé avec un enduit comprenant de la cellulose microcristalline et de la carraghénane.

31. Procédé de préparation de la composition pharmaceutique comprenant les étapes suivantes :
a) la préparation d'un mélange de substance active sélectionnée à partir du groupe qui comprend de l'acide alendronique, ses esters ou sels, un ou plusieurs charges et un ou plusieurs excipients pharmaceutiquement acceptables sélectionnés à partir du groupe comprenant des délitants, des glissants et autres excipients pharmaceutiquement acceptables,
b) le mélange mélangé avec un lubrifiant,
c) la compression du mélange résultant en compacts,
d) la mouture des compacts en granules,
e) la préparation du mélange de granules et d'un lubrifiant,
f) la compression du mélange résultant en comprimés,
g) le pelliculage du comprimé avec un enduit comprenant de la cellulose microcristalline et de la carraghénane.

32. Procédé de préparation de la composition pharmaceutique comprenant les étapes suivantes :
a) la préparation d'un mélange de substance active sélectionnée à partir du groupe qui comprend de l'acide alendronique, ses esters ou sels, un ou plusieurs charges et un ou plusieurs excipients pharmaceutiquement acceptables sélectionnés à partir du groupe comprenant des délitants, des glissants et autres excipients pharmaceutiquement acceptables,
b) la préparation de granules par la méthode de granulation humide,
c) la préparation du mélange de granules et d'un lubrifiant,
d) la compression du mélange résultant en comprimés,
e) le pelliculage du comprimé avec un enduit comprenant de la cellulose microcristalline et de la carraghénane.

33. Procédé de préparation de la composition pharmaceutique comme revendiqué aux revendications 30 à 32, **caractérisé en ce que** les compositions pharmaceutiques comprennent de 5 à 280 mg d'acide alendronique ou d'un de ses sels ou esters pharmaceutiquement acceptables, sur base du poids actif d'acide alendronique.

34. Procédé pour préparer la composition pharmaceutique selon l'une des revendications 30 à 33, **caractérisé en ce que** la composition pharmaceutique du noyau du comprimé se désagrégeant rapidement comprend uniquement une charge.

35. Procédé pour préparer la composition pharmaceutique selon l'une des revendications 30 à 34, **caractérisé en ce que** la charge est choisi à partir d'un groupe comprenant de la cellulose microcristalline, de la cellulose microcristalline silicifiée, de la cellulose en poudre, du lactose, du mannitol, du sorbitol, du phosphate de calcium et du phosphate d'hydrogène de calcium.

36. Procédé pour préparer la composition pharmaceutique selon la revendication 35, **caractérisé en ce que** la charge est de la cellulose microcristalline.

37. Procédé pour préparer la composition pharmaceutique selon la revendication 35, **caractérisé en ce que** la charge est de la cellulose microcristalline silicifiée.

38. Procédé pour préparer la composition pharmaceutique selon l'une des revendications 30 à 37, **caractérisé en ce que** le délitant est sélectionné à partir du groupe qui comprend de la croscarmellose sodique, de l'amidon, de l'amidon prégélatinisé, du carboxyméthyl amidon sodique, de la carboxyméthylcellulose calcique, de la carboxyméthylcellulose sodique, de l'hydroxypropylcellulose faiblement substituée, de acide alginique, de l'acide pectinique, de la polyvinylpyrrolidone, du dioxyde de silicium fortement dispersé et de l'alginate sodique.

39. Procédé pour préparer la composition pharmaceutique selon la revendication 38, **caractérisé en ce que** le délitant est de la croscarmellose sodique.

40. Procédé pour préparer la composition pharmaceutique selon l'une des revendications 30 à 39, **caractérisé en ce que** le glissant est sélectionné à partir du groupe qui comprend du dioxyde de silicium fortement dispersé, du talc, du stéarate de magnésium, du stéarate de calcium, du stéarate d'aluminium, de l'acide palmitique, de l'acide stéarique, du stéarol, du cétanol et du polyéthylène glycol.

41. Procédé pour préparer la composition pharmaceutique selon la revendication 40, **caractérisé en ce que** le glissant est du dioxyde de silicium.

42. Procédé pour préparer la composition pharmaceutique selon l'une des revendications 30 à 41, **caractérisé en ce que** le lubrifiant est sélectionné à partir du groupe qui comprend de l'acide stéarique, du stéarate de magnésium, du stéarate de calcium, du stéarate d'aluminium, du stéarate de zinc, du laurylsulfate de sodium, du stéaryl-fumarate de sodium, du talc, du talc siliconisé, de l'huile végétale hydrogénée et des polyéthylène glycols.

43. Procédé pour préparer la composition pharmaceutique selon la revendication 42, **caractérisé en ce que** le lubrifiant est du stéarate de magnésium.

44. Procédé pour préparer la composition pharmaceutique selon l'une des revendications 30 à 43, **caractérisé en ce que** le pelliculage, en plus de la cellulose microcristalline et de la carraghénane, comprend un ou plusieurs excipients pharmaceutiquement acceptables sélectionnés à partir du groupe qui comprend des polymères de renforcement, des plastifiants, des diluants, des agents tensio-actifs, des colorants et des amplificateurs de brillance.
